# EUROPEAN PATENT APPLICATION

(11) **EP 4 062 977 A1**
(43) Date of publication of application: **28.09.2022**
(21) Application number: 20890318.7
(22) Date of filing: 19.11.2020
(51) Int. Cl.: A61Q 1/14, C11D 1/10, C11D 1/74, C11D 1/88, C11D 1/92, C11D 3/18, C11D 3/20, A61K 8/02, A61K 8/31, A61K 8/37, A61K 8/44, A61K 8/46, A61K 8/49, A61K 8/86, A61K 8/891, A61K 8/92, A61K 8/9789

(54) **DETERGENT COMPOSITION FOR FOAMER**

(30) Priority: 20.11.2019 JP 2019209772
(71) Applicant: AJINOMOTO CO., INC., Chuo-ku Tokyo 104-8315 (JP)
(72) Inventor: IKEDA, Naoaki, Kawasaki-shi, Kanagawa 210-8681 (JP)
(74) Representative: Mewburn Ellis LLP
(86) International application number: PCT/JP2020/043216
(87) International publication number: WO 2021/100812

(57) **Abstract**

The present invention relates to a cleansing composition for a foamer, containing (A) an anionic surfactant, (B) a non-ionic surfactant having an HLB value of 7 to 11 and liquid at 25°C, (C) an amphoteric surfactant, (D) polyhydric alcohol, (E) oil, and (F) water, wherein a ratio of the content of component (A) and the content of component (B) [(A)/(B)] (mass ratio) is 0.4 to 3.8, and a ratio of the content of component (B) and the content of component (E) [(B)/(E)] (mass ratio) is 0.4 to 6, the composition having a viscosity of not more than 500 mPa·s at 25°C and a pH of 3 to 6 at 25°C.

According to the present invention, a cleansing composition for a foamer can be provided, which composition is superior in the cleansing off power on makeup cosmetics and foamability, and also superior in the discharge property from a foamer container. Furthermore, a cleansing composition for a foamer, permitting preferable use of an acyl acidic amino acid or a salt thereof as a surfactant can be provided.

## Description

### [Technical Field]

The present invention relates to a cleansing composition for a foamer. The cleansing composition is suitable for use with a foamer container.

### [Background Art]

A cleansing composition for washing the face is used in order to wash away stains such as sebum, dust, and the like on the face, makeup cosmetics, and the like and obtain healthy skin.

Makeup cosmetics contain oily components, powders, and polymer compounds, and it is difficult to wash them off with general cleansing compositions. Therefore, a nonionic surfactant or an oily component is often used as the main component in cleansing compositions aiming at removing and cleansing off makeup cosmetics.

However, nonionic surfactants are poor in foam volume and foam quality, and it is very difficult to obtain a comfortable texture and a comfortable feeling of use due to the rich foaming desired by the consumers. Furthermore, there is a problem that the use of an oily component in a detergent composition in order to improve the performance of cleansing off makeup cosmetics further decreases the foamability.

While anionic surfactants are also widely used as foaming surfactants in cleansing compositions for body and face, they are poor in the ability to solubilize oily components. Therefore, there is a problem that they show a low ability to remove makeup cosmetics, and decrease the stability of dissolved state when used in a composition containing an oily component.

Furthermore, in recent years, the type of cleaning agent that is discharged as foam from a foamer container has been attracting attention because of its convenience. In order to discharge a cleansing composition as foam, it is necessary to control the property of the cleansing composition so that the nozzle of the foamer container will not be clogged.

In view of the above-mentioned problems, attempts have been made to improve a cleansing composition in terms of the foamability, foam quality, cleansing power, and foam discharge property from a foamer container.

For example, as a makeup remover composition that can be discharged as long-lasting foam by a non-aerosol pump type foam dispenser, a composition containing straight chain fatty acid soap, acylglycinate, acylarginine, water, and polyhydric alcohol, each in a specific amount, has been proposed (Patent Literature 1).

The makeup remover composition described in Patent Literature 1 is a highly transparent aqueous cleansing composition scarcely containing a water-insoluble skin softener. However, since it does not contain oily components, the power to cleanse off makeup cosmetics may be insufficient.

As a cleansing composition improved in both the cleansing power and foamability, a cleansing composition containing a non-ionic surfactant having a branched alkyl group or two or more alkyl or alkenyl groups and an HLB value of 3 to 12, an oil which is liquid at room temperature, an anionic surfactant with ethylene oxide chain, a polyhydric alcohol having a specific IOB value, and an inorganic salt (Patent Literature 2), and a foamable composition in the form of a nanoemulsion or microemulsion and containing an oil, a polyglyceryl fatty acid ester having an HLB value of 8 to 13, an alkyl(poly)glycoside, an amphoteric surfactant, and water (Patent Literature 3) have been proposed.

The cleansing composition described in Patent Literature 2 has a cleansing power capable of sufficiently removing oily mascara and is a transparent composition. The composition characteristically foams during rinsing and is not presupposed to be used with a foamer container.

The foamable composition described in Patent Literature 3 is a composition in the form of a nanoemulsion or a microemulsion which is a thermodynamically stable isotropic single liquid phase, and a transparent or semi-transparent composition. Its pH was shown to be 6.5 to 7.0 (Examples). The present inventor found that, in a cleansing composition for a foamer, containing an oil and an anionic surfactant as a main surfactant, the transparency of the composition decreases when the pH of the composition exceeds 6, and the nozzle is likely to be clogged.

On the other hand, acyl acidic amino acids or salts thereof such as N-acylglutamic acid salt have a mild action on the skin and hair, are superior in biodegradability, and have a foaming action, a cleaning action, a penetrating action, an emulsifying action, an antistatic action, and an antibacterial action. Therefore, it is an anionic surfactant desired to be used also in the compositions used for cleansing off makeup cosmetics.

However, as mentioned above, for stable use in a foamer container, it is necessary to control the pH of the cleansing composition to 6 or less. As a result, a cleansing composition containing an acyl acidic amino acid or a salt thereof has a problem that the uniformity of the composition decreases at a low pH, thus making its use in a foamer container difficult.

Therefore, the development of a cleansing composition that is suitable for use in a foamer container and also permits use of an acyl acidic amino acid or a salt thereof has been desired.

### [Citation List]

### [Patent Literature]

[PTL 1]
   JP-A-2015-526451
[PTL 2]
   JP-A-2012-140364
[PTL 3]
   JP-A-2014-122196

### [Summary of Invention]

### [Technical Problem]

Accordingly, the present invention aims to provide a cleansing composition for a foamer, which composition is superior in the cleansing off power on makeup cosmetics and foamability, and also superior in the discharge property from a foamer container. The present invention further aims to provide a cleansing composition for a foamer, which composition can preferably utilize an acyl acidic amino acid or a salt thereof as a surfactant.

### [Solution to Problem]

The present inventor conducted intensive studies in an attempt to solve the above-mentioned problem and found that the above-mentioned problem can be solved by using (A) an anionic surfactant, (B) a specific non-ionic surfactant, (C) an amphoteric surfactant, (D) polyhydric alcohol, (E) oil, and (F) water, setting the content ratio of component (A) and component (B) [(A)/(B)] (mass ratio) and the content ratio of component (B) and component (E) [(B)/(E)] (mass ratio) to each fall within a specific range, and further adjusting the viscosity and pH of the cleansing composition to each fall within a specific range, and completed the present invention.

Accordingly, the present invention relates to the following.
[1] A cleansing composition for a foamer, comprising (A) an anionic surfactant, (B) a non-ionic surfactant having an HLB value of 7 to 11 and liquid at 25°C, (C) an amphoteric surfactant, (D) polyhydric alcohol, (E) oil, and (F) water, wherein a ratio of the content of component (A) and the content of component (B) [(A)/(B)] (mass ratio) is 0.4 to 3.8, and a ratio of the content of component (B) and the content of component (E) [(B)/(E)] (mass ratio) is 0.4 to 6, the composition having a viscosity of not more than 500 mPa·s at 25°C and a pH of 3 to 6 at 25°C.
[2] The cleansing composition of [1], wherein the (A) anionic surfactant is acyl acidic amino acid or a salt thereof.
[3] The cleansing composition of [1] or [2], wherein the (B) non-ionic surfactant having an HLB value of 7 to 11 and liquid at 25°C is polyoxyethylene glycerol fatty acid ester.
[4] The cleansing composition of any of [1] to [3], wherein the (C) amphoteric surfactant is one or more kinds selected from the group consisting of a sulfobetaine-type amphoteric surfactant and an imidazoline-type amphoteric surfactant.
[5] The cleansing composition of any of [1] to [4], wherein the (E) oil is one or more members selected from the group consisting of a hydrocarbon oil, an ester oil, and saturated fatty acid triglyceride, and liquid at 25°C.
[6] The cleansing composition of any of [1] to [5], wherein a content of the (E) oil is 0.01 mass %-6 mass %.
[7] The cleansing composition of any of [1] to [6], wherein the viscosity at 25°C is not more than 100 mPa·s.
[8] The cleansing composition of any of [1] to [7], having a transmittance of not less than 70% at a measurement wavelength of 430 nm.
[9] The cleansing composition of any of [1] to [8], wherein the composition is filled in a foamer container.

### [Advantageous Effects of Invention]

According to the present invention, a cleansing composition for a foamer can be provided, which composition is superior in the cleansing off power on makeup cosmetics and foamability, and also superior in the discharge property from a foamer container.

According to the present invention, moreover, a cleansing composition for a foamer, which composition stably contains an acyl acidic amino acid or a salt thereof, has a mild action on the skin, and is superior in biodegradability, antibacterial action, and the like can be provided.

### [Description of Embodiments]

The cleansing composition for a foamer of the present invention (hereinafter also to be referred to as "the composition of the present invention" in the present specification) has the following characteristics (i) to (iii):
(i) the composition contains (A) an anionic surfactant, (B) a non-ionic surfactant having an HLB value of 7 to 11 and liquid at 25°C, (C) an amphoteric surfactant, (D) polyhydric alcohol, (E) oil, and (F) water,
(ii) a content ratio of component (A) and component (B) [(A)/(B)] (mass ratio) is 0.4 to 3.8, and a content ratio of component (B) and component (E) [(B)/(E)] (mass ratio) is 0.4 to 6,
(iii) the composition has a viscosity of not more than 500 mPa·s at 25°C and a pH of 3 to 6 at 25°C.

In the present specification, the "cleansing composition for a foamer" refers to a cleansing composition suitable for filling and providing in a foamer container mentioned below.

The anionic surfactant contained as component (A) in the composition of the present invention is a surfactant in which a hydrophilic group ionizes into anion in an aqueous solution, and can be used without particular limitation as long as the characteristics of the present invention are not impaired.

Examples thereof include carboxylate salts such as fatty acid soap, polyoxyethylene alkyl ether carboxylate, acyl lactate, N-acylamino acid salt and the like; salts of sulfonic acid such as alkane sulfonate, α-olefin sulfonate, α-sulfone fatty acid methyl ester salt, and the like; sulfuric ester salts such as alkyl sulfuric acid ester salt, secondary alkyl sulfuric acid ester salt, alkyl ether sulfuric acid ester salt, monoacylglycerol sulfuric acid ester salt, and the like; phosphoric acid ester salts such as alkyl phosphoric acid ester salt, polyoxyethylene alkyl ether phosphoric acid ester salt, and the like; and the like, and one or more members can be used.

From the aspects of a mild action on the skin, high safety, and superiority in biodegradability, N-acylamino acid salts such as N-acyl-sarcosine salt, N-acylmethylalanine salt, N-acylacidic amino acid salt (N-acylglutamic acid salt, N-acylaspartic acid salt, etc.), and the like are preferably used as the anionic surfactant. As described above, an N-acylacidic amino acid salt is more preferably used because it is superior in a foaming action, a washing action, a penetration action, an emulsifying action, an antistatic action, and an antibacterial action, and shows a particularly mild action on the skin.

As the N-acylacidic amino acid salt, an N-acylaspartic acid salt and an N-acylglutamic acid salt are recited as examples, and N-acylglutamic acid salt is preferred, and N-acyl-L-glutamic acid salt is more preferred.

In addition, one having, as the acyl group, an acyl group derived from a saturated or unsaturated fatty acid having 8-22 carbon atoms is preferred. Examples of such acyl group include octanoyl (capryloyl), 2-ethylhexanoyl, nonanoyl, decanoyl (caprinoyl), undecanoyl, undecenoyl, dodecanoyl (lauroyl), tetradecanoyl (myristoyl), hexadecanoyl (palmitoyl), octadecanoyl (stearoyl), 2-heptylundecanoyl (isostearoyl), (Z)-9-octadecenoyl (oleoyl), (9Z,12Z)-octadeca-9,12-dienoyl (linoleoyl), (9Z,12Z,15Z)-octadeca-9,12,15-trienoyl (α-linolenoyl), icosanoyl (arachidoyl), docosanoyl (behenoyl), and the like. In addition, acyl groups derived from mixed fatty acids derived from natural fats and oils such as coconut oil, palm kernel oil, and the like (coconut oil fatty acid acyl, palm kernel oil fatty acid acyl, etc.) are also preferred.

For the purpose of the present invention, one having an acyl group having 8-18 carbon atoms is more preferably used, and one having an acyl group having 10-16 carbon atoms is further preferably used.

As the salt, an alkali metal salt such as sodium salt, potassium salt or the like; an alkanol amine salt such as triethanolamine salt or the like, a basic amino acid salt such as lysine salt, arginine salt, histidine salt or the like, or the like is preferably used.

Specifically, sodium N-lauroyl-L-aspartate, potassium N-lauroyl-L-aspartate, triethanolamine N-lauroyl-L-aspartate, disodium N-lauroyl-L-aspartate, dipotassium N-lauroyl-L-aspartate, sodium N-myristoyl-L-aspartate, potassium N-myristoyl-L-aspartate, triethanolamine N-myristoyl-L-aspartate, disodium N-myristoyl-L-aspartate, dipotassium N-myristoyl-L-aspartate, sodium N-palmitoyl-L-aspartate, potassium N-palmitoyl-L-aspartate, triethanolamine N-palmitoyl-L-aspartate, disodium N-palmitoyl-L-aspartate, dipotassium N-palmitoyl-L-aspartate, sodium N-stearoyl-L-aspartate, potassium N-stearoyl-L-aspartate, triethanolamine N-stearoyl-L-aspartate, disodium N-stearoyl-L-aspartate, dipotassium N-stearoyl-L-aspartate, sodium N-coconut oil fatty acid acyl-L-aspartate, potassium N-coconut oil fatty acid acyl-L-aspartate, N-coconut oil fatty acid acyl-L-aspartic acid triethaolamine, disodium N-coconut oil fatty acid acyl-L-aspartate, dipotassium N-coconut oil fatty acid acyl-L-aspartate, sodium N-palm kernel oil fatty acid acyl-L-aspartate, potassium N-palm kernel oil fatty acid acyl-L-aspartate, N-palm kernel oil fatty acid acyl-L-aspartic acid triethanolamine, disodium N-palm kernel oil fatty acid acyl-L-aspartate, dipotassium N-palm kernel oil fatty acid acyl-L-aspartate, sodium N-lauroyl-L-glutamate, potassium N-lauroyl-L-glutamate, triethanolamine N-lauroyl-L-glutamate, disodium N-lauroyl-L-glutamate, dipotassium N-lauroyl-L-glutamate, sodium N-myristoyl-L-glutamate, potassium N-myristoyl-L-glutamate, triethanolamine N-myristoyl-L-glutamate, disodium N-myristoyl-L-glutamate, dipotassium N-myristoyl-L-glutamate, sodium N-palmitoyl-L-glutamate, potassium N-palmitoyl-L-glutamate, triethanolamine N-palmitoyl-L-glutamate, disodium N-palmitoyl-L-glutamate, dipotassium N-palmitoyl-L-glutamate, sodium N-stearoyl-L-glutamate, potassium N-stearoyl-L-glutamate, triethanolamine N-stearoyl-L-glutamate, disodium N-stearoyl-L-glutamate, dipotassium N-stearoyl-L-glutamate, sodium N-coconut oil fatty acid acyl-L-glutamate, potassium N-coconut oil fatty acid acyl-L-glutamate, N-coconut oil fatty acid acyl-L-glutamic acid triethanolamine, disodium N-coconut oil fatty acid acyl-L-glutamate, dipotassium N-coconut oil fatty acid acyl-L-glutamate, sodium N-palm kernel oil fatty acid acyl-L-glutamate, potassium N-palm kernel oil fatty acid acyl-L-glutamate, N-palm kernel oil fatty acid acyl-L-glutamic acid triethanolamine, disodium N-palm kernel oil fatty acid acyl-L-glutamate, dipotassium N-palm kernel oil fatty acid acyl-L-glutamate and the like, and sodium N-lauroyl-L-glutamate, potassium N-lauroyl-L-glutamate, triethanolamine N-lauroyl-L-glutamate, disodium N-lauroyl-L-glutamate, dipotassium N-lauroyl-L-glutamate, sodium N-coconut oil fatty acid acyl-L-glutamate, potassium N-coconut oil fatty acid acyl-L-glutamate, N-coconut oil fatty acid acyl-L-glutamic acid triethanolamine, disodium N-coconut oil fatty acid acyl-L-glutamate, dipotassium N-coconut oil fatty acid acyl-L-glutamate, and the like are particularly preferably used.

In the present invention, the above-mentioned anionic surfactant may be produced by a production method known per se, or commercially available products provided by each company can also be used.

The content of component (A) in the composition of the present invention is generally 1 mass % to 25 mass %, preferably 1 mass % to 20 mass %, more preferably 1 mass % to 15 mass %.

The composition of the present invention contains a non-ionic surfactant having an HLB (Hydrophilic-Lipophilic Balance) value of 7 to 11 and liquid at 25°C as component (B).

Examples of such non-ionic surfactant include polyglycerol fatty acid esters such as hexaglyceryl monomyristate, decaglyceryl diisostearate, decaglyceryl trioleate and the like; polyoxyethylene alkyl ethers such as polyoxyethylene(2E.O.) lauryl ether, polyoxyethylene(3E.O.) alkyl(12-14) ether, polyoxyethylene(4.2E.O.) lauryl ether, polyoxyethylene(5E.O.) alkyl(12-14) ether, and the like; polyoxyethylene alkenyl ethers such as polyoxyethylene(2E.O.) oleyl ether, polyoxyethylene(7E.O.) oleyl ether, and the like; polyoxyethylene polyoxypropylene alkyl ethers such as polyoxyethylene(1E.O.) polyoxypropylene(4P.O.) cetyl ether and the like; polyoxyethylene sorbitol fatty acid esters such as polyoxyethylene(6E.O.) sorbitol tetraoleate, polyoxyethylene(30E.O.) sorbitol tetraoleate, and the like; polyethylene glycol fatty acid esters such as polyethylene glycol(6E.O.) monooleate, polyethylene glycol(10E.O.) monooleate, and the like; polyoxyethylene sorbitan fatty acid esters such as polyoxyethylene(6E.O.) sorbitan monostearate, polyoxyethylene(6E.O.) sorbitan monooleate, polyoxyethylene(20E.O.) sorbitan trioleate, and the like; polyoxyethylene hydrogenated castor oils such as polyoxyethylene(20E.O.) hydrogenated castor oil, polyoxyethylene(30E.O.) hydrogenated castor oil, and the like; polyoxyethylene glycerol fatty acid esters such as polyoxyethylene(20E.O.) glyceryl triisostearate, polyoxyethylene(40E.O.) glyceryl triisostearate, and the like, and the like, and one or more members can be used.

For the purpose of the present invention, polyoxyethylene hydrogenated castor oil, polyoxyethylene sorbitol fatty acid ester, polyoxyethylene glycerol fatty acid ester, and the like are preferably used, and polyoxyethylene glycerol fatty acid ester is particularly preferably used.

In the present invention, the above-mentioned non-ionic surfactant may be produced by a production method known per se, or commercially available products provided by each company can also be used.

The content of component (B) in the composition of the present invention is generally 1 mass % to 25 mass %, preferably 1 mass % to 20 mass %, more preferably 1 mass % to 15 mass %.

The amphoteric surfactant contained as component (C) in the composition of the present invention can be used without particular limitation as long as it is a surfactant having both an anionic group and a cationic group in a molecule and the characteristics of the present invention are not impaired.

Examples thereof include glycine-type amphoteric surfactants such as alkylglycine salt, alkylcarboxymethylglycine salt, and the like; imidazoline-type amphoteric surfactants such as N-acylaminoethyl-N-2-hydroxyethylglycine salt (sodium N-lauroyl-N'-carboxymethyl-N'-hydroxyethylethylenediamine, sodium N-coconut oil fatty acid acyl-N'-carboxymethyl-N'-hydroxyethylethylenediamine, etc.) and the like; hydroxyalkyl(C12-14) hydroxyethyl sarcosine; aminopropionic acid-type amphoteric surfactants such as alkylaminopropionic acid salt, alkyliminodipropionic acid salt, N-acylaminoethyl-N-2-hydroxyethylpropionic acid salt (sodium N-coconut oil fatty acid acyl-N'-carboxyethyl-N'-hydroxyethylethylenediamine etc.), and the like; aminoacetic acid betaine-type amphoteric surfactants such as alkyl dimethylaminoacetic acid betaine (lauryl dimethylaminoacetic acid betaine, coconut oil alkyl dimethylaminoacetic acid betaine etc.), fatty acid amide propyl dimethylaminoacetic acid betaine (lauroyl amide propyl dimethylaminoacetic acid betaine, coconut oil fatty acid amide propyl dimethylaminoacetic acid betaine, palm kernel oil fatty acid amide propyl dimethylaminoacetic acid betaine, etc.), alkyl dihydroxyethylamino acetic acid betaine, and the like; sulfobetaine-type amphoteric surfactants such as N-alkyl-N,N-dimethylammonium-N-propylsulfonic acid salt, N-alkyl-N,N-dimethylammonium-N-(2-hydroxypropyl)sulfonic acid salt, fatty acid amide propyl-N,N-dimethylammonium-N-(2-hydroxypropyl)sulfonic acid salt (lauroyl amide propyl-N,N-dimethylammonium-N-(2-hydroxypropyl)sulfonate, etc.), and the like; amineoxide-type amphoteric surfactants such as alkyldimethylamineoxide (lauryl dimethylamineoxide, etc.) and the like; phosphoric acid-type amphoteric surfactants such as phosphatidyl choline and the like; and the like, and one or more members can be used.

For the purpose of the present invention, sulfobetaine-type amphoteric surfactant and imidazoline-type amphoteric surfactant are preferably used, sodium N-coconut oil fatty acid acyl-N'-carboxymethyl-N'-hydroxyethylethylenediamine, sodium N-lauroyl-N'-carboxymethyl-N'-hydroxyethylethylenediamine, lauroyl amide propyl-N,N-dimethylammonium-N-(2-hydroxypropyl) sulfonic acid salt, coconut oil fatty acid amide propyl-N,N-dimethylammonium-N-(2-hydroxypropyl)sulfonic acid salt, and the like are more preferably used.

In the present invention, the above-mentioned amphoteric surfactant may be produced by a production method known per se, or commercially available products provided by each company can also be used.

The content of component (C) in the composition of the present invention is generally 1 mass % to 25 mass %, preferably 1 mass % to 20 mass %, more preferably 3 mass % to 20 mass %.

The polyhydric alcohol contained as component (D) in the composition of the present invention can be used without particular limitation as long as it is an alcohol having two or more hydroxyl groups in a molecule. Examples thereof include diols such as propylene glycol, dipropylene glycol, 1,3-butylene glycol, 1,3-propanediol, pentylene glycol, hexylene glycol, polyethylene glycol, and the like; triols such as glycerol and the like; sugar alcohols such as xylitol, sorbitol, maltitol, and the like, and the like. One or more members of these can be used.

For the purpose of the present invention, diol or triol having 3-6 carbon atoms is preferably used, and propylene glycol, dipropylene glycol, 1,3-butylene glycol, glycerol and the like are more preferably used.

In the present invention, the above-mentioned polyhydric alcohol may be produced by a production method known per se, or commercially available products provided by each company can also be used.

The content of component (D) in the composition of the present invention is generally 1 mass % to 20 mass %, preferably 1 mass % to 15 mass %, more preferably 1 mass % to 12 mass %.

The oil contained as component (E) in the composition of the present invention is a hydrophobic substance that is collected from animals, plants, minerals, etc. and phase-separated from water, and an oil that is liquid at 25°C is preferably used.

Examples thereof include hydrocarbon oils such as liquid paraffin, liquid isoparaffin, light liquid isoparaffin, squalane, and the like; ester oils such as cetyl 2-ethylhexanoate, isononyl isononanoate, isotridecyl isononanoate, isopropyl myristate, isopropyl palmitate, 2-ethylhexyl palmitate, octyldodecyl myristate, isopropyl isostearate, neopentylglycol dicaprate, diisostearyl malate, isopropyl N-lauroylsarcosinate, di(phytosteryl/2-octyldodecyl) N-lauroyl-L-glutamate, di(phytosteryl/behenyl/2-octyldodecyl) N-lauroyl-L-glutamate, and the like; saturated fatty acid triglyceride such as glyceryl tri(2-ethylhexanoate), glyceryl tri(caprylate/caprate), glyceryl triisostearate, and the like; vegetable oils such as almond oil, avocado oil, olive oil, rice germ oil, soybean oil, evening primrose oil, rape seed oil, sunflower oil, grapes seed oil, macadamia nut oil, and the like; plant waxes such as jojoba oil and the like; cyclic silicone oils such as decamethylcyclopentasiloxane, octamethylcyclotetrasiloxane, and the like; silicone oils such as dimethylpolysiloxane, methylphenylpolysiloxane, and the like; and the like. One or more of these members can be used.

From the aspects of the cleansing off power on makeup cosmetics, mixability in a composition, obtainability, and the like, hydrocarbon oil, ester oil, and saturated fatty acid triglyceride which are liquid at 25°C are more preferably used, and isopropyl N-lauroylsarcosinate, isopropyl myristate, glyceryl tri(caprylate/caprate), and the like are further preferably used.

In the present invention, the above-mentioned oil may be collected or extracted from respective animals, plants, minerals, etc. and purified, or may be produced by a production method known per se, or commercially available products provided by each company can also be used.

The content of component (E) in the composition of the present invention is generally 0.01 mass % to 10 mass %, preferably 0.01 mass % to 6 mass %, more preferably 0.03 mass % to 6 mass %.

The composition of the present invention contains water as component (F). Examples of water include purified water such as distillated water, deionized water, and the like, tap water, industrial water, and the like. Water suitable for the production of cosmetics, quasi-drugs, pharmaceutical products, and the like is used.

The content of water is set to an amount with respect to the total amount of the composition of the present invention as 100 mass %.

In the composition of the present invention, the content ratio of the above-mentioned component (A) and the above-mentioned component (B) [(A)/(B)] (mass ratio) is 0.4 to 3.8, preferably 0.4 to 3.0, more preferably 0.4 to 2.5.

In addition, the content ratio of the above-mentioned component (B) and the above-mentioned component (E) [(B)/(E)] (mass ratio) is 0.4 to 6, preferably 0.4 to 5, more preferably 0.4 to 2.

When the content ratio of component (A) and component (B) [(A)/(B)] is less than 0.4 or more than 3.8, or when the content ratio of component (B) and component (E) [(B)/(E)] is less than 0.4 or more than 6, the uniformity of the composition tends to decrease, which is not preferable because the nozzle of a foamer container may be clogged.

Furthermore, in order to improve the discharge property from a foamer container, the composition of the present invention is preferably a liquid having low viscosity. In the composition of the present invention, the viscosity at 25°C is not more than 500 mPa·s, preferably not more than 200 mPa·s, more preferably not more than 100 mPa·s.

When the viscosity at 25°C exceeds 500 mPa·s, the discharge of the composition of the present invention from a foamer container becomes difficult and the usability is degraded.

The viscosity at 25°C can be determined by measuring at 25°C by a Brookfield rotational viscometer using rotor No. 1 or No. 2 and setting the rotation speed to 6 rpm, 12 rpm, or 60 rpm.

The pH of the composition of the present invention at 25°C is 3-6, preferably 3.2-5.8, more preferably 3.2-5.0, further preferably 3.5-4.8, still more preferably 3.8-4.8.

When the pH of the cleansing composition is less than 3, skin irritation may occur during rinsing. When the pH of the cleansing composition exceeds 6, the composition may become non-uniform, the appearance may be deteriorated, the nozzle of a foamer container may be clogged, and the discharge property may be degraded.

The pH can be measured by the glass electrode method.

The composition of the present invention exhibits a uniform and good appearance, and is preferably transparent. The transmittance of the composition of the present invention at 430 nm is preferably not less than 70%, more preferably not less than 80%.

The composition of the present invention may contain, in addition to component (A) to component (F), general additives used in cosmetics, quasi-drugs, pharmaceutical products, and the like, as long as the characteristics of the present invention are not impaired.

Examples of such additive include lower alcohols such as ethanol, isopropanol and the like; surfactants other than components (A) to (C); humectants such as sodium pyrrolidone carboxylate and the like; thickening polysaccharides such as xanthan gum and the like; pH adjusters such as citric acid, malic acid, potassium hydroxide, sodium hydroxide, sodium carbonate, and the like; antiseptics such as p-hydroxybenzoic acid ester, phenoxyethanol, and the like; chelating agents such as sodium ethylenediamine tetraacetate and the like; antioxidants such as vitamin E and the like; plant extracts such as aloe extract, tea extract, and the like; sebum inhibitor; anti-inflammatory agent; dye; flavor, and the like.

One or more members of these additives may be contained as necessary. The content of these additives can be determined according to the amount generally contained in cleansing compositions.

The composition of the present invention can be produced according to a conventional method.

For example, the composition can be prepared by sequentially adding component (A), component (C), component (D) and other hydrophilic additives as necessary to component (F), mixing them, adjusting pH by adding a pH adjuster as necessary to give an aqueous phase component, gradually adding the aqueous phase component with stirring to an oil phase component prepared by mixing component (B), component (E) and other lipophilic additives as necessary, and uniformly mixing them.

The composition of the present invention is superior in the cleansing off power on makeup cosmetics and in foamability, and is also superior in the discharge property from a foamer container.

Therefore, the composition of the present invention is suitable for use by filling in a foamer container.

The foamer container is a non-gas type foam discharge container in which a cleansing composition and air are mixed in a predetermined ratio inside the container, and the mixture is passed through one or more porous bodies with a given pore size, or plastic or metal mesh, which is embedded in the nozzle head, and discharged in the form of foam.

As the non-gas type foam discharge container, a squeeze foamer container that discharges foam by manually squeezing the bottle barrel part, a pump foamer container that discharges foam by pushing down the nozzle, and the like can be mentioned. A container made from plastic such as high-density polyethylene, polypropylene, polyethylene terephthalate, or the like is preferably used.

As such foamer container, containers manufactured by Daiwa Can Co., Ltd., Yoshino Kogyosho Co., Ltd., and the like can be used.

Therefore, the present invention also provides a cleansing composition in the form of being filled in a foamer container.

As the foamer container, both the above-mentioned squeeze foamer container and the pump foamer container can be used.

The cleansing composition to be filled in a foamer container is as described above with respect to the composition of the present invention.

The amount of the cleansing composition to be filled in a foamer container is preferably 50% to 90%, more preferably 60% to 80%, of the volume of the foamer container.

The cleansing composition of the present invention in the above-mentioned form suppresses the occurrence of nozzle clogging, and the cleansing composition can be discharged from the foamer container as good and stable foam.

### [Example]

The present invention is further explained in detail by referring to Examples.

According to the formulations shown in Table 1 to 4 and by the method shown below, the cleansing compositions of Examples and Comparative Examples were prepared.

### <Production method>

The components (A), (C), (D) and other components in the Tables were added to component (F) and mixed uniformly, and the pH was adjusted to give aqueous phase components. The pH in the Tables shows a value measured by the glass electrode method in a room set to 25°C. On the other hand, components (B) and (E) in the Tables were uniformly mixed to give an oil phase component. The aforementioned aqueous phase component was added with stirring to the oil phase component and uniformly mixed.

The prepared respective cleansing compositions of Examples and Comparative Examples were evaluated for the appearance and cleansing power of the compositions and the viscosity was measured in a room set to 25°C.

### (1) Evaluation of appearance

Using ultraviolet visible near infrared spectrophotometer V-570 (manufactured by JASCO Corporation), the respective cleansing compositions of Examples and Comparative Examples were filled in 1 cm square disposable cells, the transmittance at a measurement wavelength of 430 nm was measured, and the appearance (transparency of the composition) was evaluated according to the following evaluation criteria.

### <evaluation criteria>

transmittance = not less than 80%: A
transmittance = not less than 30%, less than 80%: B
transmittance = less than 30%: C

### (2) Evaluation of cleansing power

A commercially available waterproof mascara was applied to a white BIOSKIN plate (manufactured by Beaulax Co., Ltd.) in an about 1 cm square. Each of the cleansing compositions (2 g) of Examples and Comparative Examples was dropped onto the center of commercially available cotton, the aforementioned cotton was folded in four, and used for an operation of wiping off the waterproof mascara on the BIOSKIN plate. The operation of wiping off the mascara was performed 10 times in a given direction by applying a force of about 200 g using an electronic analytical scale. Then, the surface of the cotton folded in four was changed and the wiping operation in the given direction was performed 10 more times. The cotton surface was further changed and the wiping operation was performed in the same manner.

The cleansing power was evaluated according to the following evaluation criteria by image evaluation of the part to which the waterproof mascara was applied.

### <Evaluation criteria>

not less than 90% of mascara was removed: A
not less than 50% and less than 90% of mascara was removed: B
not less than 20% and less than 50% of mascara was removed: C
less than 20% of mascara was removed :D

### (3) Measurement of viscosity at 25°C

Using a Brookfield type viscometer (manufactured by Tokyo Keiki Inc.), rotor No. 1 or No. 2 was used according to the viscosity region of the cleansing composition to be measured, and the viscosity was measured at the rotation speed of 6 rpm, 12 rpm, or 60 rpm. Each of the cleansing compositions of Examples and Comparative Examples was filled in a 100 mL plastic container and subjected to the measurement.

### (4) Comprehensive evaluation

When an evaluation of A was obtained in both the evaluation of appearance and the evaluation of cleansing power, the comprehensive evaluation was A; when the evaluation of A was obtained in any of them, the comprehensive evaluation was B; and when the evaluation of A was not obtained in any of them, the comprehensive evaluation was C.

The evaluation results and the measurement results of (1) to (4) are also shown in each of Tables 1 to 4.

Based on the evaluation results and the measurement results of (1) to (4), the influence of each of the pH, the viscosity, the content ratio of component (A) and component (B) [(A)/(B)], the content ratio of component (B) and component (E) [(B)/(E)], and the kind of components (A) to (E) on the property and function of the cleansing compositions was examined.

### [Examination of influence of pH of cleansing composition]

The composition and evaluation results of the cleansing compositions of Examples 1 to 5 and Comparative Example 1 prepared by adjusting the pH of the composition to a different pH are shown in Table 1.

In the Table, the content of sodium N-coconut oil fatty acid acyl-L-glutamate indicates the net content of the component.

**[Table 1]**

| component | | name of starting material (supplier) | Ex. 1 | Ex. 2 | Ex. 3 | Ex. 4 | Ex. 5 | Com. Ex. 1 |
|---|---|---|---|---|---|---|---|---|
| (A) | sodium N-coconut oil fatty acid acyl-L-glutamate | AMISOFT CS-22 (25 mass %) (Ajinomoto Co., Inc.) | 2.5 | 2.5 | 2.5 | 2.5 | 2.5 | 2.5 |
| (B) | polyoxyethylene(20E.O.) glyceryl triisostearate (HLB value = 8) | GWIS-320EX (Nihon Emulsion Co., Ltd.) | 2.0 | 2.0 | 2.0 | 2.0 | 2.0 | 2.0 |
| (C) | lauroyl amide propyl-N,N-dimethyl ammonium-N-(2-hydroxypropyl) sulfonate | Softazoline LSB (Kawaken Fine Chemicals Co., Ltd.) | 3.5 | 3.5 | 3.5 | 3.5 | 3.5 | 3.5 |
| (D) | dipropylene glycol | DPG-RF (ADEKA Corporation) | 5.0 | 5.0 | 5.0 | 5.0 | 5.0 | 5.0 |
| (E) | isopropyl myristate | IPM-R (Kokyu Alcohol Kogyo Co., Ltd.) | 2.0 | 2.0 | 2.0 | 2.0 | 2.0 | 2.0 |
| (F) | ion-exchanged water | purified water | amount based on total amount as 100 mass % | | | | | |
| others | 5 mass % potassium hydroxide aqueous solution | potassium hydroxide [reagent grade] (FUJIFILM Wako Pure Chemical Corporation) | appropriate amount | | | | | |
| | 10 mass % aqueous citric acid solution | citric acid (Fuso Chemical Co., Ltd.) | appropriate amount | | | | | |
| pH | | | 4.5 | 3.5 | 5.0 | 5.5 | 6.0 | 7.0 |
| (A)/(B) | | | 1.25 | 1.25 | 1.25 | 1.25 | 1.25 | 1.25 |
| (B)/(E) | | | 1.00 | 1.00 | 1.00 | 1.00 | 1.00 | 1.00 |
| appearance | | | A | A | B | C | C | C |
| viscosity at 25°C (mPa·s) | | | 9.5 | 9.7 | 5.0 | 4.1 | 3.8 | 4.2 |
| cleansing power | | | A | A | A | A | A | D |
| comprehensive evaluation | | | A | A | B | B | B | C |

As shown in Table 1, each cleansing composition of Examples 1 to 3 having a pH of 3.5 to 5.0 exhibited a good appearance with high transparency or slightly high transparency, and showed good cleansing power. Each cleansing composition of Examples 4 and 5 having a pH of 5.5 and 6.0, respectively, showed a decrease in the transparency of the appearance but the cleansing power was good.

On the other hand, the cleansing composition of Comparative Example 1 having a pH of 7.0 showed a decrease in the transparency of the appearance and also a decrease in the cleansing power.

From the above evaluation results, it was suggested that the pH of the composition at 25°C should be not more than 6 from the aspects of the uniformity and cleansing power of the cleansing composition.

### [Examination of influence of kind and viscosity at 25°C of component (C)]

The composition and evaluation results of the cleansing compositions of Examples 1, 3, 6, 7 and Comparative Examples 2 and 3 prepared using different kinds of amphoteric surfactants as component (C) are shown in Table 2.

In the Table, the content of sodium N-coconut oil fatty acid acyl-L-glutamate indicates the net content of the component. The blank column in the Table means that the component was not contained.

**[Table 2]**

| component | | name of starting material (suppl ier) | Ex. 1 | Ex. 3 | Ex. 6 | Ex. 7 | Com. Ex. 2 | Com. Ex. 3 |
|---|---|---|---|---|---|---|---|---|
| (A) | sodium N-coconut oil fatty acid acyl-L-glutamate | AMISOFT CS-22 (25 mass %) (Ajinomoto Co., Inc.) | 2.5 | 2.5 | 2.5 | 2.5 | 2.5 | 2.5 |
| (B) | polyoxyethylene(20E.O.) glyceryl triisostearate (HLB value = 8) | GWIS-320EX (Nihon Emulsion Co., Ltd.) | 2.0 | 2.0 | 2.0 | 2.0 | 2.0 | 2.0 |
| (C) | lauroyl amide propyl-N,N-dimethylammonium-N-(2-hydroxypropyl) sulfonate | Softazoline LSB (Kawaken Fine Chemicals Co., Ltd.) | 3.5 | 3.5 | | | | |
| | sodium N-coconut oil fatty acid acyl-N'-carboxymethyl-N'-hydroxyethylethylenediamine | Softazoline CH (Kawaken Fine Chemicals Co., Ltd.) | | | 3.0 | | | |
| | coconut oil alkyldimethylaminoacetic acid betaine | GENAGEN B 1566 (Clariant AG) | | | | 3.0 | | |
| | coconut oil fatty acid amide propyldimethylaminoacetic acid betaine | AMPHITOL 55AB (Kao Corporation) | | | | | 3.0 | |
| | lauroyl amide propyldimethylaminoacetic acid betaine | Softazoline LPB-R (Kawaken Fine Chemicals Co., Ltd.) | | | | | | 3.0 |
| (D) | dipropylene glycol | DPG-RF (ADEKA Corporation) | 5.0 | 5.0 | 5.0 | 5.0 | 5.0 | 5.0 |
| (E) | isopropyl myristate | IPM-R (Kokyu Alcohol Kogyo Co., Ltd.) | 2.0 | 2.0 | 2.0 | 2.0 | 2.0 | 2.0 |
| (F) | ion-exchanged water | purified water | amount based on total amount as 100 mass % | | | | | |
| others | 5 mass % potassium hydroxide aqueous solution | potassium hydroxide [reagent grade] (FUJIFILM Wako Pure Chemical Corporation) | appropriate amount | | | | | |
| | 10 mass % aqueous citric acid solution | citric acid (Fuso Chemical Co., Ltd.) | appropriate amount | | | | | |
| pH | | | 4.5 | 5.0 | 5.0 | 4.5 | 5.0 | 5.0 |
| (A)/(B) | | | 1.25 | 1.25 | 1.25 | 1.25 | 1.25 | 1.25 |
| (B)/(E) | | | 1.00 | 1.00 | 1.00 | 1.00 | 1.00 | 1.00 |
| appearance | | | A | B | A | A | C | C |
| viscosity at 25°C (mPa·s) | | | 9.5 | 5.0 | 4.0 | 4.4 | 1095 | 2056 |
| cleansing power | | | A | A | C | C | D | D |
| comprehensive evaluation | | | A | B | B | B | C | C |

As shown in Table 2, each cleansing composition of Examples 1 and 3 containing a sulfobetaine-type amphoteric surfactant as component (C) exhibited a good appearance with high transparency or slightly high transparency, and showed good cleansing power.

The cleansing composition of Example 6 containing an imidazoline-type amphoteric surfactant as component (C) and having a pH of 5.0, and the cleansing composition of Example 7 containing alkyl dimethylaminoacetic acid betaine as component (C) and having a pH of 4.5 showed high transparency but showed a slight decrease in the cleansing power.

On the other hand each cleansing composition of Comparative Examples 2 and 3 containing fatty acid amide propyl dimethylaminoacetic acid betaine as component (C) exhibited a viscosity exceeding 500 mPa·s at 25°C, which is a high viscosity not suitable for use in a foamer container, and showed a decrease in the transparency of appearance and the cleansing power.

### [Examination of influence of content ratio of component (A) and component (B) and content ratio of component (B) and component (E)]

The composition and evaluation results of each of the cleansing compositions of Examples 1, 8 to 15, and Comparative Examples 4 to 6 prepared by changing the content ratio of component (A) and component (B) [(A)/(B)] and the content ratio of component (B) and component (E) [(B)/(E)] are shown in Table 3.

In the Table, the content of sodium N-coconut oil fatty acid acyl-L-glutamate indicates the net content of the component

**[Table 3]**

| component | | name of starting material (supplier) | Ex. 1 | Ex. 8 | Ex. 9 | Ex. 10 | Ex. 11 | Ex. 12 | Ex. 13 | Ex. 14 | Ex. 15 | Com. Ex. 4 | Com. Ex. 5 | Com. Ex. 6 |
|---|---|---|---|---|---|---|---|---|---|---|---|---|---|---|
| (A) | sodium N-coconut oil fatty acid acyl-L-glutamate | AMISOFT CS-22 (25 mass %) (Ajinomoto Co., Inc.) | 2.5 | 2.5 | 3 | 2.5 | 3.5 | 2.5 | 2.5 | 2.5 | 2.5 | 4.0 | 2.5 | 2.5 |
| (B) | polyoxyethylene(20E. O.) glyceryl triisostearate (HLB value = 8) | GWIS-320EX (Nihon Emulsion Co., Ltd.) | 2.0 | 2.5 | 1.5 | 3.0 | 1.0 | 2.5 | 3.0 | 4.0 | 5.0 | 1.0 | 7.0 | 7.0 |
| (C) | lauroyl amide propyl-N,N-dimethylammonium-N-(2-hydroxypropyl)-sulfonate | Softazoline LSB (Kawaken Fine Chemicals Co., Ltd.) | 3.5 | 3.5 | 3.5 | 3.5 | 3.5 | 3.5 | 3.5 | 3.5 | 3.5 | 3.5 | 3.5 | 3.5 |
| (D) | dipropylene glycol | DPG-RF (ADEKA Corporation) | 5.0 | 5.0 | 5.0 | 5.0 | 5.0 | 10.0 | 10.0 | 10.0 | 10.0 | 10.0 | 10.0 | 10.0 |
| (E) | isopropyl myristate | IPM-R (Kokyu Alcohol Kogyo Co., Ltd.) | 2.0 | 1.5 | 2.0 | 1.0 | 2.0 | 2.5 | 3.0 | 4.0 | 5.0 | 1.0 | 1.0 | 7.0 |
| (F) | ion-exchanged water | purified water | amount based on total amount as 100 mass % | | | | | | | | | | | |
| others | 5 mass % potassium hydroxide aqueous solution | potassium hydroxide [reagent grade] (FUJIFILM Wako Pure Chemical Corporation) | appropriate amount | | | | | | | | | | | |
| | 10 mass % aqueous citric acid solution | citric acid (Fuso Chemical Co., Ltd.) | appropriate amount | | | | | | | | | | | |
| pH | | | 4.5 | 4.5 | 4.5 | 4.5 | 4.5 | 4.5 | 4.5 | 4.5 | 4.5 | 4.5 | 4.5 | 4.5 |
| (A)/(B) | | | 1.25 | 1.00 | 2.00 | 0.83 | 3.5 | 1.00 | 0.83 | 0.63 | 0.50 | 4.00 | 0.36 | 0.36 |
| (B)/(E) | | | 1.00 | 1.67 | 0.75 | 3.00 | 0.50 | 1.00 | 1.00 | 1.00 | 1.00 | 1.00 | 7.00 | 1.00 |
| appearance | | | A | A | A | A | C | A | A | A | A | C | C | C |
| viscosity at 25°C (mPa·s) | | | 9.5 | 9.5 | 9.1 | 9.5 | 4.6 | 4.0 | 4.3 | 4.7 | 13.0 | 19.0 | 21.0 | 4.2 |
| cleansing power | | | A | A | A | B | A | A | A | A | A | B | B | B |
| comprehensive evaluation | | | A | A | A | B | B | A | A | A | A | C | C | C |

As shown in Table 3, each of the cleansing compositions of Examples 1, 8 to 15 in which the content ratio of component (A) and component (B) [(A)/(B)] was 0.50 to 3.5, and the content ratio of component (B) and component (E) [(B)/(E)] was 0.50 to 3.00 achieved the comprehensive evaluation of A or B.

On the other hand, the cleansing composition of Comparative Example 6 in which the content ratio of component (A) and component (B) [(A)/(B)] was less than 0.4, the cleansing composition of Comparative Example 5 in which the aforementioned [(A)/(B)] was less than 0.4, and the content ratio of component (B) and component (E) [(B)/(E)] was more than 6, and the cleansing composition of Comparative Example 4 in which the aforementioned [(A)/(B)] was more than 3.8 showed a decrease in the transparency of appearance and the comprehensive evaluation was C.

### [Examination of influence of kind of components (A) to (E)]

The composition and evaluation results of each of the cleansing compositions of Examples 1, 16 to 29, and Comparative Example 7 prepared by respectively changing the kind of the anionic surfactant as component (A), the kind of the non-ionic surfactant as component (B) and the HLB value, the kind of the amphoteric surfactant as component (C), the kind of the polyhydric alcohol as component (D), the kind of the oil as component (E), and the kind of other components are shown in Table 4.

In the Table, the content of each of sodium N-coconut oil fatty acid acyl-L-glutamate, sodium α-olefin (C14-16)sulfonate, and sodium pyrrolidone carboxylate indicates the net content of the component. The blank column in the Table means that the component was not contained.

**[Table 4]**

| component | | name of starting material (supplier) | Ex. 1 | Ex. 16 | Ex. 17 | Ex. 18 | Ex. 19 | Ex. 20 | Ex. 21 | Ex. 22 | Ex. 23 | Ex. 24 | Ex. 25 | Ex. 26 | Ex. 27 | Ex. 28 | Ex. 29 | Com. Ex. 7 |
|---|---|---|---|---|---|---|---|---|---|---|---|---|---|---|---|---|---|---|
| (A) | sodium N-coconut oil fatty acid acyl-L-glutamate | AMISOFT CS-22 (25 mass %) (Ajinomoto Co., Inc.) | 2.5 | | | 2.5 | 2.5 | 2.5 | 2.5 | 2.5 | 2.5 | 2.5 | 2.5 | 2.5 | 2.5 | 2.5 | 2.5 | 2.5 |
| | sodium polyoxyethylene lauryl ether sulfate | EMAL E-27C (Kao Corporation) | | 2.5 | | | | | | | | | | | | | | |
| | sodium α-olefin (C14-16) sulfonate | LIPOLAN LB-440 (37 mass %) (Lion Corporation) | | | 2.5 | | | | | | | | | | | | | |
| (B) | polyoxyethylene(20E.O.) glyceryl triisostearate (HLB value = 8) | GWIS-320EX (Nihon Emulsion Co., Ltd.) | 2.0 | 2.0 | 2.0 | | 2.0 | 2.0 | 2.0 | 2.0 | 2.0 | 2.0 | 2.0 | 2.0 | 2.0 | 2.0 | 2.0 | |
| | polyoxyethylene(40E.O.) glyceryl triisostearate (HLB value = 11) | GWIS-340EX (Nihon Emulsion Co., Ltd.) | | | | 2.0 | | | | | | | | | | | | |
| | polyoxyethylene(20E.O.) glyceryl isostearate (HLB value = 13) | GWIS-120 (Nihon Emulsion Co., Ltd.) | | | | | | | | | | | | | | | | 2.0 |
| (C) | lauroyl amide propyl-N, N-dimethylammonium-N-(2-hydroxypropyl) sulfonate | Softazoline LSB (Kawaken Fine Chemicals Co., Ltd.) | 3.5 | 3.5 | 3.5 | 3.5 | 3.5 | 3.5 | 3.5 | 3.5 | 3.5 | 3.5 | 3.5 | 3.5 | 3.5 | 2.8 | 3.5 | 3.5 |
| | coconut oil fatty acid amide propyldimethylaminoacetic acid betaine | AMPHITOL 55AB (Kao Corporation) | | | | | | | | | | | | | | 0.6 | | |
| (D) | dipropylene glycol | DPG-RF (ADEKA Corporation) | 5.0 | 5.0 | 5.0 | 5.0 | | | 5.0 | 5.0 | 5.0 | 5.0 | 5.0 | 5.0 | 5.0 | 5.0 | 5.0 | 5.0 |
| | glycerol | concentrated glycerin (Kao Corporation) | | | | | 5.0 | | | | | | | | | | | |
| | propylene glycol | propylene glycol for cosmetics (ADEKA Corporation) | | | | | | 5.0 | | | | | | | | | | |
| (E) | isopropyl myristate | IPM-R (Kokyu Alcohol Kogyo Co., Ltd.) | 2.0 | 2.0 | 2.0 | 2.0 | 2.0 | 2.0 | | | | 2.0 | 2.0 | 2.0 | 1.5 | 2.0 | 1.5 | 2.0 |
| | liquid paraffin | P-55 (MORESCO Corporation) | | | | | | | 2.0 | | | | | | | | | |
| | jojoba oil | jojoba oil (Nikko Chemicals Co., Ltd.) | | | | | | | | 2.0 | | | | | | | | |
| | macadamia nut oil | macadamia nut oil (Nikko Chemicals Co., Ltd.) | | | | | | | | | 2.0 | | | | | | | |
| | decamethylcyclopentasiloxane | SH-245 (Dow Corning Toray Co., Ltd.) | | | | | | | | | | | | | 0.5 | | | |
| | glyceryl tri(caprylate/caprate) | TCG-M (Kokyu Alcohol Kogyo Co., Ltd.) | | | | | | | | | | | | | | | 0.5 | |
| (F) | ion-exchanged water | purified water | amount based on total amount as 100 mass % | | | | | | | | | | | | | | | |
| others | polyoxyethylene(20E.O.) sorbitan monooleate (HLB value = 15) | TO-10V (Nikko Chemicals Co., Ltd.) | | | | | | | | | | 2.0 | | | | | | |
| | phenoxyethanol | phenoxyethanol-S (Yokkaichi Chemical Company Limited) | | | | | | | | | | | 1.0 | | | | | |
| | sodium pyrrolidone carboxylate | AJIDEW NL-50 (50 mass %) (Ajinomoto Co., Inc.) | | | | | | | | | | | | 2.0 | 2.0 | 2.0 | | |
| | 5 mass % potassium hydroxide aqueous solution | potassium hydroxide [reagent grade] (FUJIFILM Wako Pure Chemical Corporation) | appropriate amount | | | | | | | | | | | | | | | |
| | 10 mass % aqueous citric acid solution | citric acid (Fuso Chemical Co., Ltd.) | appropriate amount | | | | | | | | | | | | | | | |
| pH | | | 4.5 | 4.5 | 4.5 | 4.5 | 4.5 | 4.5 | 4.5 | 4.5 | 4.5 | 4.5 | 4.5 | 4.5 | 4.5 | 4.5 | 4.5 | 4.5 |
| (A)/(B) | | | 1.25 | 1.25 | 1.25 | 1.25 | 1.25 | 1.25 | 1.25 | 1.25 | 1.25 | 1.25 | 1.25 | 1.25 | 1.25 | 1.25 | 1.25 | 1.25 |
| (B)/(E) | | | 1.00 | 1.00 | 1.00 | 1.00 | 1.00 | 1.00 | 1.00 | 1.00 | 1.00 | 1.00 | 1.00 | 1.00 | 1.33 | 1.00 | 1.33 | 1.00 |
| appearance | | | A | B | B | A | A | A | A | C | C | A | A | A | A | A | A | C |
| viscosity at 25°C (mPa·s) | | | 9.5 | 3.7 | 4.3 | 4.6 | 13.0 | 8.0 | 5.0 | 11.0 | 11.0 | 4.4 | 4.7 | 4.2 | 4.7 | 4.2 | 4.7 | 4.2 |
| cleansing power | | | A | A | B | B | A | B | C | A | A | A | B | B | B | B | B | C |
| comprehensive evaluation | | | A | B | B | B | A | B | B | B | B | A | B | B | B | B | B | C |

As shown in Table 4, each of the cleansing compositions of Examples 1, 16 to 29 achieved the comprehensive evaluation of A or B.

Each of the cleansing compositions of Example 22 containing jojoba oil, which is a plant-derived wax, as component (E), and Example 23 containing macadamia nut oil, which is a vegetable oil, as component (E) showed a decrease in the transparency of the appearance. In addition, the cleansing composition of Example 21 containing liquid paraffin, which is a hydrocarbon oil, as component (E) showed a decrease in the cleansing power. From such results, it was suggested that the use of ester oil and saturated fatty acid triglyceride as component (E) is preferable from the aspects of transparency and cleansing power of the cleansing composition.

The cleansing composition of Comparative Example 7 containing a non-ionic surfactant having an HLB value of 13 as component (B) showed a decrease in the transparency of the appearance and a decrease in the cleansing power, and the comprehensive evaluation was C.

On the other hand, the cleansing composition of Example 24 containing a hydrophilic (HLB value = 15) non-ionic surfactant in addition to a non-ionic surfactant having an HLB value of 8 as component (B) showed high transparency and good cleansing power. Thus, it was suggested that a hydrophilic non-ionic surfactant can be used in addition to component (B).

### [Industrial Applicability]

As described in detail above, according to the present invention, a cleansing composition for a foamer can be provided, which composition is superior in the cleansing off power on makeup cosmetics and foamability, is also superior in the discharge property from a foamer container, and is suitable for use in a foamer container.

According to the present invention, moreover, a cleansing composition for a foamer, which composition stably contains an acyl acidic amino acid or a salt thereof, has a mild action on the skin, and is superior in biodegradability, antibacterial action, and the like can be provided.

Also, according to the present invention, a cleansing composition in the form of being filled in a foamer container, which suppresses the occurrence of nozzle clogging, and achieves discharge of the cleansing composition from the foamer container as good and stable foam can be provided.

This application is based on a patent application No. 2019-209772 filed in Japan, the contents of which are incorporated in full herein.

## Claims

1. A cleansing composition for a foamer, comprising (A) an anionic surfactant, (B) a non-ionic surfactant having an HLB value of 7 to 11 and liquid at 25°C, (C) an amphoteric surfactant, (D) polyhydric alcohol, (E) oil, and (F) water, wherein a ratio of the content of component (A) and the content of component (B) [(A)/(B)] (mass ratio) is 0.4 to 3.8, and a ratio of the content of component (B) and the content of component (E) [(B)/(E)] (mass ratio) is 0.4 to 6, the composition having a viscosity of not more than 500 mPa·s at 25°C and a pH of 3 to 6 at 25°C.

2. The cleansing composition according to claim 1, wherein the (A) anionic surfactant is acyl acidic amino acid or a salt thereof.

3. The cleansing composition according to claim 1 or 2, wherein the (B) non-ionic surfactant having an HLB value of 7 to 11 and liquid at 25°C is polyoxyethylene glycerol fatty acid ester.

4. The cleansing composition according to any one of claims 1 to 3, wherein the (C) amphoteric surfactant is one or more kinds selected from the group consisting of a sulfobetaine-type amphoteric surfactant and an imidazoline-type amphoteric surfactant.

5. The cleansing composition according to any one of claims 1 to 4, wherein the (E) oil is one or more members selected from the group consisting of a hydrocarbon oil, an ester oil, and saturated fatty acid triglyceride, and liquid at 25°C.

6. The cleansing composition according to any one of claims 1 to 5, wherein a content of the (E) oil is 0.01 mass %-6 mass %.

7. The cleansing composition according to any one of claims 1 to 6, wherein the viscosity at 25°C is not more than 100 mPa·s.

8. The cleansing composition according to any one of claims 1 to 7, having a transmittance of not less than 70% at a measurement wavelength of 430 nm.

9. The cleansing composition according to any one of claims 1 to 8, wherein the composition is filled in a foamer container.
